# EUROPEAN PATENT SPECIFICATION

(11) **EP 0 692 261 B1**
(45) Date of publication and mention of the grant of the patent: **03.05.2000**
(21) Application number: 95304763.6
(22) Date of filing: 07.07.1995
(51) Int. Cl.: A61L 2/08, A61L 2/12, A61L 2/06

(54) **Sterilizing method and apparatus**
Verfahren und Vorrichtung zur Sterilisation
Procédé et appareil de stérilisation

(30) Priority: 12.07.1994 JP 16001394
(43) Date of publication of application: 17.01.1996
(73) Proprietor: Eisai Co., Ltd., Tokyo (JP)
(72) Inventor: Shimizu, Katsumi, Honjo-shi, Saitama-ken (JP); Honda, Washiro, Honjo-shi, Saitama-ken (JP)
(74) Representative: Rackham, Stephen Neil

(56) References cited:
- EP-A- 0 355 425
- EP-A- 0 401 775
- EP-A- 0 496 633
- DE-A- 2 029 792
- FR-A- 2 166 973
- FR-A- 2 547 732
- US-A- 3 674 422
- US-A- 3 880 586

## Description

The present invention relates to a method of consecutively carrying out heat sterilization of a sealed vessel such as a vial having a plug such as a rubber stopper and containing a medical liquid such as a medicine or vaccine and, more particularly, to an apparatus for carrying out the method.

Conventionally, sterilization treatment of a vial containing a medical liquid such as injection material has been conducted as part of the production process. A method of steam sterilization using a high pressure steam tank is well known. However, steam sterilization is a method batch process and the temperature of each vial has not been able to be measured individually.

Consequently, each vial has not been independently detected whether the sterilization is completely carried out and a guarantee for the sterilization has had no choice but to depend on the condition of the whole batch.

Furthermore, it has not been possible to carry out measurement of the temperature of the inside of the plug, so that it has been difficult to guarantee that it is certainly sterilized.

However, in the method of sterilizing vials consecutively, the inside of their plugs should be sterilized to the same level as the medical liquid.

A method of sterilizing vials consecutively by heating from the outside using hot air is conventional but this does not heat to a temperature enough to completely sterilize the inside of the plug because of an aluminium cap or a resin flip cap covering the plug. The resin flip cap can also be varied and destroyed by the application of hot air from the outside.

The purposes of the present invention are met by solving the aforesaid art disadvantages and by providing a sterilizing apparatus and method capable of sequentially carrying out heat sterilization of a whole sealed vessel, such as a vial, having an inside plug and so on and liquid medicine held therein for a short time and also by a relatively simple structure.

The concrete method for achieving the purposes of the present invention will be illustrated below.

Accordingly, in a first aspect, the invention consists in a sterilizing method for sterilizing a sealed vessel, such as a vial, having a plug, such as a rubber stopper, during sequential conveyance, comprising the steps of:
moving each sealed vessel while tilting it until a medical liquid in the sealed vessel touches the inside of the plug and rotating the sealed vessel;
heating the medical liquid in the tilted sealed vessel by heat energy selected from infra-red line heating, gas heating and microwave heating;
measuring the temperature of the medical liquid in each of the sealed vessels; and
maintaining the sealed vessel at the predetermined temperature for a certain period of time so that a heat sterilization of the whole of the inside of the sealed vessel including the inside of the plug is carried out by means of the heated medical liquid.

In the aforesaid method, it is advisable that the sealed vessel should be cooled and dried after remaining at the predetermined temperature for a certain period of time.

The sterilization can be assured by measuring a temperature of the liquid medicine in each of the sealed vessel by a thermometer (for example, an infrared radiation thermometer) after the temperature of the sealed vessel reaches a predetermined temperature, automatically controlling the operating condition of the infrared heater, gas heating or microwave heating corresponding to the changing temperature, and defining a heating temperature of the liquid medicine of each sealed vessel.

The concrete apparatus for achieving the purposes of the present invention will be illustrated below.

In a further aspect, the invention consists in a sterilizing apparatus for sterilization treatment of a sealed vessel, such as a vial, having a plug, such as a rubber stopper, during sequential conveyance, comprising:
a device for holding the sealed vessel in a tilted position with a medical liquid touching the inside of the plug during the conveying process;
a device causing the sealed vessel to move and simultaneously rotate whilst tilted;
a heating device selected from an infra-red line heater, gas heater and microwave heater to heat the medical liquid in the sealed vessel whilst it is tilted,
a thermometer for measuring the liquid temperature in the sealed vessel; and
a heat retaining device such as a heater to maintain the temperature of the sealed vessel for a certain period of time after the sealed vessel reaches a predetermined temperature to sterilize the medical liquid and thereby the whole of the inside of the sealed vessel.

It is advisable that the sterilizing apparatus includes a cooling device and drying device next to the heat-retaining device to maintain for a certain period of time in the temperature after the sealed vessel reaches predetermined temperature.

The sterilization can be assured by the sterilizing apparatus including a control system to automatically control the heating condition of the sealed vessel, which heating condition relatives to the changes in the temperature of liquid medicine in each sealed vessel by measuring with the infrared radiation thermometer provided at the end of a heating portion during the conveying process in order to maintain predetermined level of the sterilized state of the liquid medicine in each sealed vessel.

Concrete operation of the present invention will be illustrated in below.

The consecutively moved plural vials in the state of being held at their necks by the conveyor bucket and being slanted at a inverting portion until the liquid medicine touches the inside plugs in the vials pass through a heating furnace such as the infra-red radiation thermometer, gas heating and microwave heating. The vial is rotated by an external belt or the like so as to be equally heated all over the whole vial including the inside plug.

The vial is retained in the heat-retaining portion such as the infrared heater and gas heating at a temperature after reaching to predetermined temperature and completely sterilized. The sterilized vial is returned to the initial upstanding state, cooled and dried in order to advance to next process.

Effectiveness of the present invention will be illustrated in below.

In the present invention, the consecutive heat sterilization of the whole sealed vessel is facilitated to equally and easily heat the temperature of the liquid medicine in the sealed vessel such as the vial having the inside plug such as the rubber stopper so that the sealed vessel is rotated and heated in slanting state until the liquid medicine in the vial touches the inside plug such as the rubber stopper.

Since output of the infrared heater, gas heating, microwave heating or the like is automatically controlled based on data which the temperature of each sealed vessel is measured by the infrared radiation thermometer provided at the heating furnace exit, although the temperature of the conveyed sealed vessel or the circumference changes, the temperature of the consecutively conveyed sealed vessel for a long time is maintained in a certain level.

The sealed vessel heated to the peak of the temperature at the heating furnace exit is retained at the heat-retaining portion provided with the heater or the like for a certain period of time so as to be completely sterilized. The sterilized sealed vessel is cooled to around room-temperature during a passage through an air-cooling portion and a water-cooling portion and then is conveyed to the next process.

The aforementioned method and apparatus in the present invention carry out the heat sterilization sequentially and certainly, regardless of the relative simple structure.

According to the method of the present invention, an autoclave will not be needed and there will be possibility of production line for process of the sealed vessel. The heat sterilization for a short time is a great advantage to facilitate to completely sterilize without any detrimental harm to a stability of temperature of the liquid medicine as compared with the autoclave.

A particular embodiment of the present invention now be described with reference to the accompanying drawings, in which:-
Fig. 1 is a partially cutaway view in perspective of the outline of the apparatus according to the present invention;
Fig. 2 is a sectional view of the inside of the heating portion (16);
Fig. 3 is a plane view of an embodiment of the device caused the vial to slant;
Fig. 4 is a side view of the device of Fig. 3;
Fig. 5 is a plane view of an embodiment of the device caused the vial to rotate; and
Fig. 6 is a front view of an example of the vial having the inside plug such as the rubber stopper.

A vial as an example of sealed vessel will be cited in the embodiments. The present invention is not intended to limit to the aforesaid example and can be used for a thing, such as a cartridge and syringe, that keeps liquid therein.

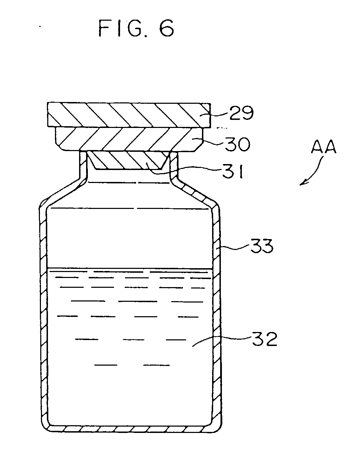

Fig. 6 illustrates the vial (AA), which a liquid medicine (32) is contained in a bottle-body (33) and the vial is sealed at its mouth with the inside plug (31) such as the rubber stopper. A numeral (30) means an aluminum-cap and a numeral (29) means a resin flip-cap in the drawing, respectively.

A discussion concerning an apparatus for sterilizing the vial (AA) in the present invention will be set forth in below.

In the apparatus of the present invention, while the vial delivered from a bucket one by one is rotating in slanted state until the medicine liquid touches the rubber stopper in the vial, the vial passes through a heating furnace such as an infrared heater, gas heating and microwave heating so as to sterilize the medicine liquid in the vial and simultaneously to effect heat sterilization of the whole vial including the rubber stopper in the vial by the sterilized medicine liquid.

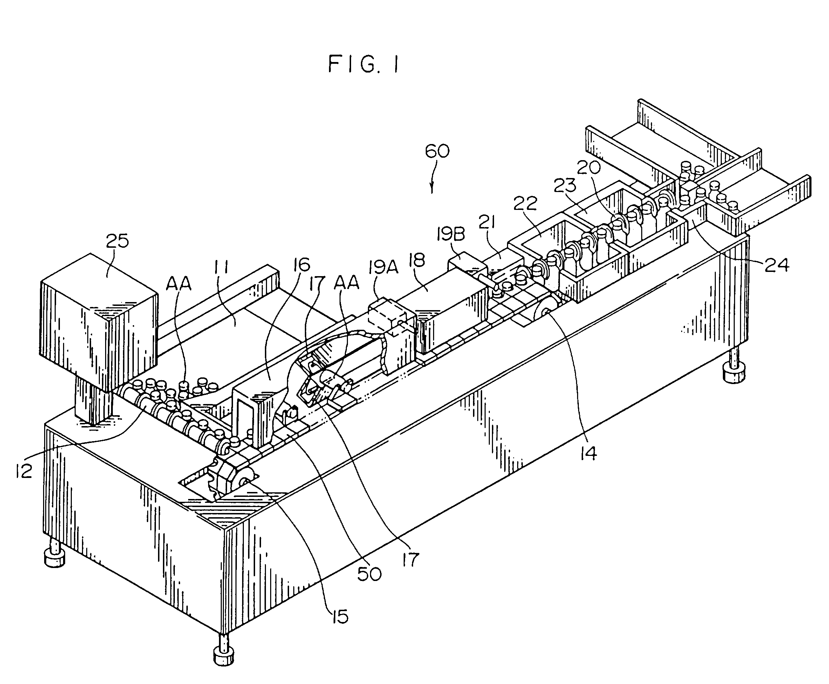

Fig. 1 is a perspective view of a sterilization device (60) according to the present invention as aforesaid.

The vial to be sterilized is taken out one by one from a feed hopper (11) by means of a screw (12) and delivered to a conveyor bucket (50) delivering the vial (AA) inside the sterilization device (60). The conveyor bucket (50) is mounted to an endless chain engaged to a pair of a driving sprocket (14) and an interlocked sub-sprocket (15).

The conveyor bucket (50) has structure varying an angle of the end of the conveyor bucket, which the vial is slanted until the medicine liquid reaches to the inside plug as soon as entering the heating furnace so as to effect the heat sterilization of the vial while passing through a heating portion (16) and a heat-retaining portion (18) in the rotating state by an external belt.

Numerals (19A) and (19B) in the drawing are of infrared radiation thermometers to measure temperature of the vial heated and sterilized in the heating portion (16) and the heat-retaining portion (18). The vial (AA) after being sterilized in the heating portion (16) and the heat-retaining portion (18) is delivered to an exited screw (20) so as to be cooled during a passage through an air-cooling portion (21) by cool air and a water-cooling portion (22) by cool water and then to be dried at a drying portion (23) by blowing air.

The vial (AA) is sorted into inferior and superior products at a distinguishing portion (24) for distinguishing whether the vial (AA) catches a temperature in a predetermined temperature limits with signals outputted from the infrared radiation thermometers (19A) and (19B) oriented at two measuring positions. Numeral (25) is of a control system, which control system (25) takes place memorization and control of the whole device about, for example, settling a sterilization temperature limits for the vial, automatically controlling output from an infrared line heater (17) and so on 17) so as to maintain a regular temperature limits for the vial by respective measuring signals send from the infrared radiation thermometers (19A) and (19B) and distinguishing whether the sterilization is completely carried out.

Next, heat sterilization process using for the infrared line heater in the heating portion (16) will be explained.

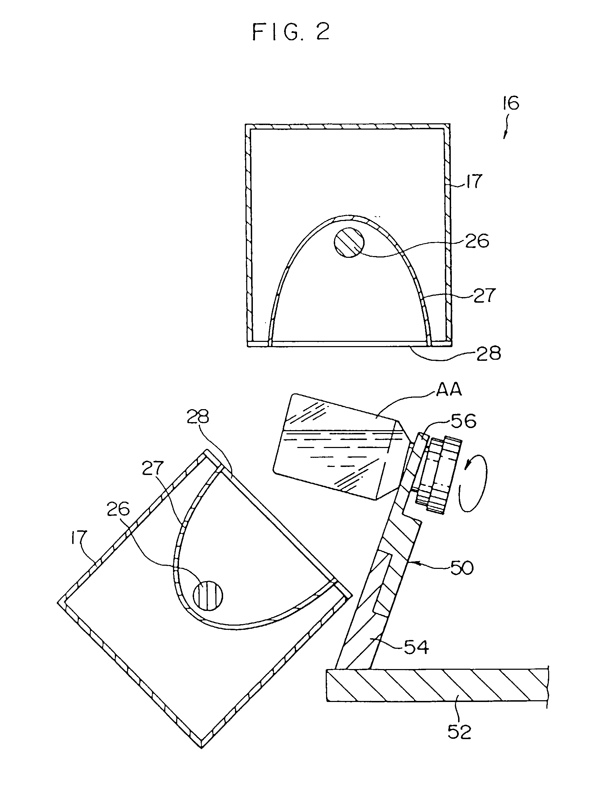

As shown in Fig. 2, the heating portion (16) heats the liquid medicine with the infrared line heater (17) adapted at the both upper and bottom direction of the vial or only the upper direction of the vial to heat the vial slanted until the inside plug in the vial touches the liquid medicine. The liquid medicine is equally heated all over because the vial is heated while being rotated by a rubber belt or the like.

The infrared line heater (17) is composed of a filament-tube (26), a converging mirror (27) and a visible radiation cut-filter (28) and can effectively heat the vial.

The visible radiation cut-filter (28) is adapted to prevent eyes of a person and the liquid medicine not standing light from damage caused by strong light.

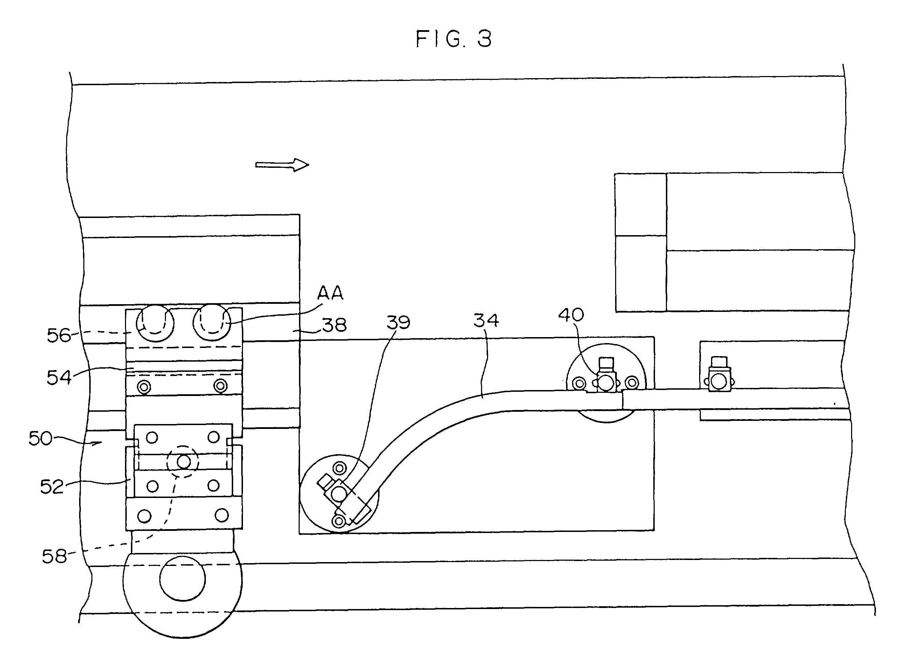

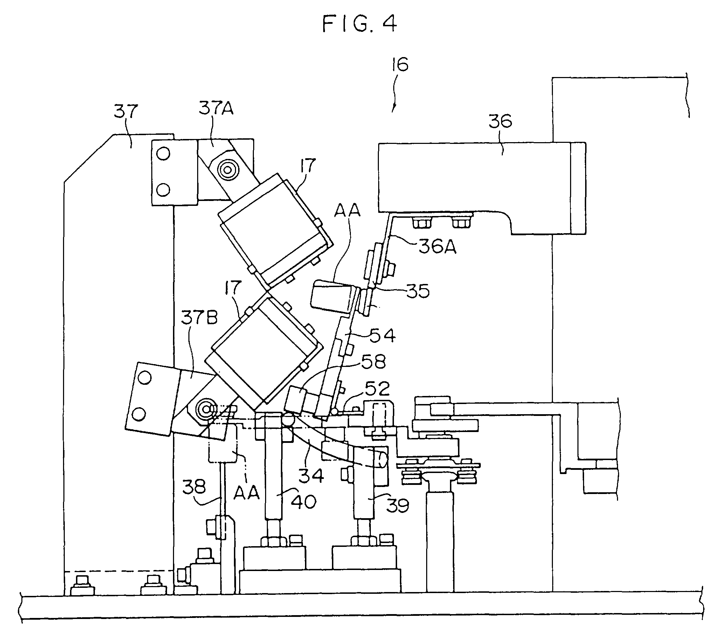

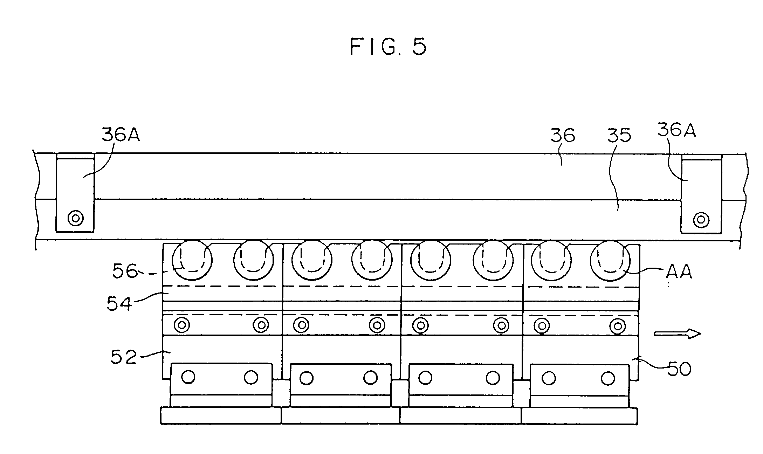

Illustrating the aforesaid device caused the vial to slant and device caused the vial to rotate in the heating portion (16), Fig. 3 and Fig. 4 shows the former device caused the vial to slant and Fig. 5 shows the later device caused the vial to rotate.

In Fig. 3 and Fig. 4, the conveyor bucket (50) is comprised of a horizontal portion (52) and a rising portion (54) movably provided thereto, which rising portion (54) has a cap portion (56) clamping the vial (AA).

The infrared line heaters (17) are oriented at stays (37A) and (37B) provided on a frame (37) of the heating portion (16) in an upward direction and a downward direction, respectively. As the vial (AA) clamped by the cap portion (56) of the conveyor bucket (50) is conveyed on a rail (38) by the conveyor bucket (50) and then comes to a cam-rail slanting bucket (34) which a cam follower (58) provided under the slanting portion (54) of the conveyor bucket (50) is supported with stands (39) and (40), the cam-rail slanting bucket (34) curves in an arcing state from the right side to the left side in Fig. 3 of a top view, namely, in an arcing state from bottom to top in Fig. 4 of a side elevational view, whereat the cam follower (58) advances corresponding to movement of the cam-rail slanting bucket (34) so as to cause the rising portion (54) to rise in a hinge portion from the horizontal portion (52) as shown in Fig. 4.

That is, an inward and upward movement of the cam-rail slanting bucket (34) attending with an advancing direction of the vial (AA) causes the cam follower (58) provided under the rising portion (54), which cam follower touches the cam-rail slanting bucket (34) with the side thereof, to be lifted slantingly and upward, so that the rising portion (54) seems to be risen along the length of the horizontal portion (52) by the hinge portion provided between the horizontal portion (52) and the rising portion (54).

The risen cap portion (56) of the conveyor bucket (50) rotates corresponding to an advance of the conveyor bucket (50) so that the vial (AA) touches a guide rotating vial (35) as shown in Fig. 5. Although the guide rotating vial (35) is supported by a metal fitting (36A) of the stay (36) in the heating portion (16), a belt can be used in stead of the guide.

As shown in Fig. 3, a rail (38) disposed near the cam-rail slanting bucket (34) is cut a position whereat the vial (AA) dose not have a need of supporting for the bottom of the vial (AA) by the rail (38) so that the vial (AA) is upward and slantingly lifted with the stands (39) and (40).

A cam-rail between the heating portion and the exited screw is adapted in the opposite direction of the cam-rail (34) in Fig. 3 to return the slanted state of the vial (AA) to the horizontal state.

Because the vial (AA) lifted slantingly and upward by the cam-rail slanting bucket (34) should be returned to an initial upstanding state.

As above stated, plural vials (AA) sequentially conveyed can be heated to around 140 °C for 30 - 40 seconds to be carried out sterilization of the whole vial and the liquid medicine therein.

The same effect is obtained if gas heating, microwave heating or the like is used as heat source in stead of the infrared line heater (17).

First the liquid medicine is heated to a certain temperature extent by the infrared line heater, gas heating, microwave heating or the like in upstanding state of the vial before being slanted, and then the heat sterilization is effected to the whole vial slanted until the liquid medicine touches the inside plug such as the rubber stopper.

Coincidentally, although Fig. 2 and 4 illustrate the slanted conveyor bucket (50) in itself in order to cause the vial (AA) to slant, other optional structure will be able to be employed.

## Claims

1. A sterilizing method for sterilizing a sealed vessel, such as a vial (AA), having a plug (31), such as a rubber stopper, during sequential conveyance, comprising the steps of:
moving each sealed vessel (AA) while tilting it until a medical liquid (32) in the sealed vessel (AA) touches the inside of the plug (31) and rotating the sealed vessel (AA);
heating the medical liquid (32) in the tilted sealed vessel (AA) by heat energy selected from infra-red line heating (26), gas heating and microwave heating;
measuring the temperature of the medical liquid (32) in each of the sealed vessels (AA); and
maintaining the sealed vessel (AA) at the predetermined temperature for a certain period of time so that a heat sterilization of the whole of the inside of the sealed vessel (AA) including the inside of the plug (31) is carried out by means of the heated medical liquid.

2. A sterilizing method according to claim 1, wherein said sealed vessel is cooled and dried after being held at the predetermined temperature for a certain period of time.

3. A sterilizing apparatus for sterilization treatment of a sealed vessel (AA), such as a vial, having a plug, such as a rubber stopper, during sequential conveyance, comprising:
a device (50) for holding the sealed vessel (AA) in a tilted position with a medical liquid (32) touching the inside of the plug during the conveying process;
a device causing the sealed vessel (AA) to move and simultaneously rotate whilst tilted;
a heating device (16) selected from an infra-red line heater, gas heater and microwave heater to heat the medical liquid (32) in the sealed vessel (AA) whilst it is tilted,
a thermometer for measuring the liquid temperature in the sealed vessel; and
a heat retaining device such as a heater (18) to maintain the temperature of the sealed vessel (AA) for a certain period of time after the sealed vessel (AA) reaches a predetermined temperature to sterilize the medical liquid and thereby the whole of the inside of the sealed vessel (AA).

4. A sterilizing apparatus according to claim 3, further comprising:
a cooling device (21, 22) and drying device (23) next to said heat retaining device (18).

5. A sterilizing apparatus according to either one of claims 3 or 4, further comprising:
a control system (25) to automatically control a heating condition of the sealed vessel (AA), which heating condition relates to the temperature of the medical liquid (32) in each sealed vessel by measuring this with an infra-red radiation thermometer (19) provided at the end of a heating portion (16, 18) during the conveying process in order to maintain a predetermined level of sterilization of the medical liquid (32) in each sealed vessel.

6. A sterilizing apparatus according to any one of claims 3 to 5, wherein the sealed vessel is conveyed by a conveyor bucket (50).

7. A sterilizing apparatus according to claim 6, wherein said device causing the sealed vessel (AA) to tilt includes a cam structure (34) provided to tilt said conveyor bucket (50) for conveying the sealed vessel (AA).

8. A sterilizing apparatus according to any one of claims 6 or 7, wherein said device causing the sealed vessel (AA) to move and simultaneously rotate in the tilted state has a means (35) for rotating the vessel (AA) as a result of its movement along the advancing direction of the conveyor bucket (50).

## Revendications

1. Procédé de stérilisation pour stériliser un récipient fermé de manière étanche, tel qu'un flacon (AA), comportant un bouchon (31), par exemple un bouchon de caoutchouc, pendant un transport successif, comprenant les étapes consistant à :
- déplacer chaque récipient (AA) fermé de manière étanche tout en le faisant basculer jusqu'à ce qu'un liquide médical (32) dans le récipient (AA) fermé de manière étanche touche l'intérieur du bouchon (31), et en faisant tourner le récipient (AA) fermé de manière étanche,
- chauffer le liquide médical (32) dans le récipient (AA) fermé de manière étanche basculé par énergie calorifique choisie parmi un chauffage (26) en ligne à rayons infrarouges, un chauffage au gaz et un chauffage aux micro-ondes,
- mesurer la température du liquide médical (32) dans chacun des récipients (AA) fermés de manière étanche, et
- conserver le récipient (AA) fermé de manière étanche à la température prédéterminée pendant une certaine plage de temps de sorte qu'une stérilisation à la chaleur de tout l'intérieur du récipient (AA) fermé de manière étanche, y compris l'intérieur du bouchon (31), soit effectuée au moyen du liquide médical chauffé.

2. Procédé de stérilisation selon la revendication 1, dans lequel ledit récipient fermé de manière étanche est refroidi et séché après avoir été conservé à la température prédéterminée pendant une certaine plage de temps.

3. Appareil de stérilisation pour le traitement de stérilisation d'un récipient (AA) fermé de manière étanche, tel qu'un flacon, comportant un bouchon, par exemple un bouchon de caoutchouc, pendant un transport successif, comprenant :
- un dispositif (50) pour retenir le récipient (AA) fermé de manière étanche dans une position inclinée contenant un liquide médical (32) en contact avec l'intérieur du bouchon pendant le processus de transport,
- un dispositif faisant se déplacer et faisant tourner simultanément le récipient (AA) fermé de manière étanche pendant qu'il est incliné,
- un dispositif de chauffage (16) choisi parmi un dispositif de chauffage en ligne à rayons infrarouges, un dispositif de chauffage au gaz et un dispositif de chauffage aux micro-ondes pour chauffer le liquide médical (32) dans le récipient (AA) fermé de manière étanche pendant qu'il est incliné,
- un thermomètre pour mesurer la température du liquide dans le récipient fermé de manière étanche, et
- un dispositif de conservation de la chaleur tel qu'un dispositif de chauffage (18) pour conserver la température du récipient (AA) fermé de manière étanche pendant une certaine plage de temps après que le récipient (AA) fermé de manière étanche a atteint une température prédéterminée pour stériliser le liquide médical et donc tout l'intérieur du récipient (AA) fermé de manière étanche.

4. Appareil de stérilisation selon la revendication 3, comprenant de plus:
- un dispositif de refroidissement (21, 22) et un dispositif de séchage (23) à la suite dudit dispositif (18) de conservation de la chaleur.

5. Appareil de stérilisation selon l'une ou l'autre des revendications 3 ou 4, comprenant de plus :
- un système de commande (25) pour commander automatiquement un état de chauffage du récipient (AA) fermé de manière étanche. lequel état de chauffage se rapporte à la température du liquide médical (32) dans chacun des récipients fermés de manière étanche, en mesurant celle-ci à l'aide d'un thermomètre (19) à rayonnement infrarouge prévu à l'extrémité d'une partie chauffante (16, 18) pendant le processus de transport afin de conserver un niveau prédéterminé de stérilisation du liquide médical (32) dans chacun des récipients fermés de manière étanche.

6. Appareil de stérilisation selon l'une quelconque des revendications 3 à 5, dans lequel le récipient fermé de manière étanche est transporté par un godet transporteur (50).

7. Appareil de stérilisation selon la revendication 6, dans lequel ledit dispositif faisant basculer le récipient (AA) fermé de manière étanche comporte une structure de came (34) prévue pour faire basculer ledit godet transporteur (50) afin de transporter le récipient (AA) fermé de manière étanche.

8. Appareil de stérilisation selon l'une quelconque des revendications 6 ou 7, dans lequel ledit dispositif amenant le récipient (AA) fermé de manière étanche à se déplacer et à tourner simultanément pour venir à l'état incliné comporte un moyen (35) pour faire tourner le récipient (AA) du fait de son déplacement dans la direction d'avance du godet transporteur (50).

## Patentansprüche

1. Verfahren zur Sterilisation eines verschlossenen Behälters (AA), insbesondere eines Fläschchens mit einem Stopfen (31), insbesondere einem Gummistopfen, während einer sequentiellen Beförderung, das folgende Schritte umfasst:
- Bewegen jeden verschlossenen Behälters (AA), wobei dieser soweit geneigt wird, dass eine medizinische Flüssigkeit (32) in dem verschlossenes Behälter die Innenseite des Stopfens (31) berührt, und Drehen des verschlossenen Behälters (AA);
- Erwärmen der medizinische Flüssigkeit (32) in dem geneigten verschlossenen Behälter (AA) mittels Heizenergie von einem Infrarotheizgerät (26), einem Gasheizgerät oder einem Mikrowellenheizgerät,
- Messen der Temperatur der medizinischen Flüssigkeit (32) in jedem verschlossenen Behälter (AA); und
- Halten des verschlossenen Behälters (AA) auf der vorbestimmten Temperatur für eine bestimme Zeitdauer, so dass über die erhitzte medizinische Flüssigkeit eine Hitzesterilisation der gesamten Innenseite des verschlossenen Behälters (AA) einschließlich der Innenseite des Stopfens (31) durchgeführt wird.

2. Sterilisationsverfahren nach Anspruch 1, dadurch gekennzeichnet, dass der verschlossene Behälter gekühlt und getrocknet wird, nachdem er für eine bestimmte Zeitdauer auf der vorbestimmten Temperatur gehalten worden ist.

3. Eine Sterilisationsvorrichtung zur Sterilisationsbehandlung eines verschlossenen Behälters (AA), insbesondere eines Fläschchens, mit einem Stopfen, insbesondere einem Gummistopfen, während sequentieller Beförderung, umfassend:
- eine Vorrichtung (50) zum Halten des verschlossene Behälters (AA) in einer geneigten Position, in welcher eine medizinische Flüssigkeit (32) während des Fördervorgangs die Innenseite des Stopfens berührt;
- eine Vorrichtung, die den verschlossene Behälter (AA) bewegt und gleichzeitig in geneigter Lage dreht;
- eine Heizvorrichtung (16), insbesondere ein Infrarotheizgerät, ein Gasheizgerät oder ein Mikrowellenheizgerät, zum Erwärmen der medizinischen Flüssigkeit (32) in dem geneigten Behälter (AA);
- ein Thermometer zum Messen der Flüssigkeitstemperatur in dem verschlossenen Behälter;
- eine Wärmerückhaltvorrichtung, insbesondere ein Heizgerät (18), um die Temperatur des verschlossenen Behälters (AA) für eine bestimmte Zeitdauer, nachdem der verschlossene Behälter (AA) eine vorbestimmte Temperatur erreicht hat, zu halten, um so die medizinische Flüssigkeit und über diese die gesamte Innenseite des verschlossenen Behälters (AA) zu sterilisieren.

4. Sterilisationsvorrichtung nach Anspruch 3, dadurch gekennzeichnet, dass sie neben der Warmerückhaltvorrichtung (18) zusätzlich eine Kühlvorrichtung (21, 22) und eine Trocknungsvorrichtung (23) umfasst.

5. Sterilisationsvorrichtung nach Anspruch 3 oder 4, gekennzeichnet durch ein Kontrollsystem (25) zur automatischen Kontrolle eines Heizzustands des verschlossenen Behälters (AA), wobei der Heizzustand von den Temperaturänderungen der medizinischen Flüssigkeit (32) in jedem verschlossenen Behälter abhängt, die durch Messung während des Fördervorgangs mit einem am Ende des Heizabschnitts (16, 18) angebrachten Infrarotstrahlungsthermometer (19) erfasst werden, um so einen vorbestimmten Sterilisationsgrad der medizinischen Flüssigkeit (32) in jedem verschlossenen Behälter zu erhalten.

6. Sterilisationsvorrichtung nach einem der Ansprüche 3 bis 5, dadurch gekennzeichnet, dass der verschlossene Behälter mit einem Förderbecher (50) befördert wird.

7. Sterilisationsvorrichtung nach Anspruch 6, dadurch gekennzeichnet, dass die Vorrichtung zum Neigen des verschlossenen Behälters (AA) eine Kurvenstruktur (34) einschließt, die vorgesehen ist, um den Förderbecher (50) für den Transport des verschlossenen Behälters (AA) zu neigen.

8. Sterilisationsvorrichtung nach Anspruch 6 oder 7, dass die Vorrichtung zum Bewegen und gleichzeitigen Drehen des verschlossenen Behälters (AA) im geneigten Zustand eine Vorrichtung (35) aufweist zum Drehen des Behälters (AA) als Resultat seiner Bewegung entlang der Förderrichtung des Förderbechers (50).
